(19) **Europäisches Patentamt / European Patent Office / Office européen des brevets**

(11) **EP 3 476 951 B1**

(12) # EUROPEAN PATENT SPECIFICATION

(45) Date of publication and mention of the grant of the patent:
**07.06.2023 Bulletin 2023/23**

(21) Application number: **18203885.1**

(22) Date of filing: **31.10.2018**

(51) International Patent Classification (IPC):
**C12Q 1/6886** (2018.01)

(52) Cooperative Patent Classification (CPC):
**C12Q 1/6886;** C12Q 2600/112; C12Q 2600/166;
C12Q 2600/178

(54) **MICRORNA-BASED METHODS AND COMPOSITIONS FOR THE DIAGNOSIS AND PROGNOSIS OF VULVAR CARCINOMA AND VULVAR INTRAEPITHELIAL LESIONS**

VERFAHREN UND ZUSAMMENSETZUNGEN AUF MIKRO-RNA-BASIS ZUR DIAGNOSE UND PROGNOSE VON VULVAKARZINOMEN UND VULVAREN INTRAEPITHELIALEN LÄSIONEN

PROCÉDÉS ET COMPOSITIONS À BASE DE MICRO-ARN POUR LE DIAGNOSTIC ET LE PRONOSTIC DE CARCINOMES DE LA VULVE ET DE LÉSIONS NÉOPLASIES INTRA-ÉPITHÉLIALES VULVAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.10.2017 EP 17460065**

(43) Date of publication of application:
**01.05.2019 Bulletin 2019/18**

(73) Proprietor: **NARODOWY INSTYTUT ONKOLOGII IM. MARII SKLODOWSKIEJ-CURIE - PANSTWOWY INSTYTUT BADAWCZY
02-781 Warszawa (PL)**

(72) Inventors:
- **KOWALEWSKA, Magdalena
  03-937 Warszawa (PL)**
- **ZALEWSKI, Kamil
  01-134 Warszawa (PL)**

(74) Representative: **LDS Lazewski Depo & Partners
ul. Prosta 70
00-838 Warsaw (PL)**

(56) References cited:
**WO-A1-2016/178519    WO-A2-2013/022786
US-A1- 2009 075 258**

- **XIUHUA YANG ET AL: "miRNA expression profile of vulvar squamous cell carcinoma and identification of the oncogenic role of miR-590-5p", ONCOLOGY REPORTS, vol. 35, no. 1, 22 October 2015 (2015-10-22), pages 398-408, XP055463468, ISSN: 1021-335X, DOI: 10.3892/or.2015.4344**
- **Beatriz Maia ET AL: "MiR-223-5p works as an oncomiR in vulvar carcinoma by TP63 suppression", Oncotarget, 9 November 2014 (2014-11-09), XP055463216, DOI: 10.18632/oncotarget.10247 Retrieved from the Internet: URL:https://www.ncbi.nlm.nih.gov/pmc/artic les/PMC5226502/pdf/oncotarget-07-49217.pdf**
- **LIEN N. HOANG ET AL: "Squamous precursor lesions of the vulva: current classification and diagnostic challenges", PATHOLOGY., vol. 48, no. 4, 20 July 2017 (2017-07-20), pages 291-302, XP055463543, AU ISSN: 0031-3025, DOI: 10.1016/j.pathol.2016.02.015**
- **DATABASE BIOSIS [Online] BIOSCIENCES INFORMATION SERVICE, PHILADELPHIA, PA, US; January 2016 (2016-01), YANG XIUHUA ET AL: "miRNA expression profile of vulvar squamous cell carcinoma and identification of the oncogenic role of miR-590-5p", Database accession no. PREV201600132023 & ONCOLOGY REPORTS, vol. 35, no. 1, January 2016 (2016-01), pages 398-408, ISSN: 1021-335X(print), DOI: 10.3892/OR.2015.4344**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Filed of disclosure**

This invention relates generally to the field of molecular biology and oncology. Certain aspects of the invention include application in diagnosis and prognosis of vulvar carcinoma and related disorders. The present disclosure provides methods pertaining to the identification of genes and gene regulators that modulate certain biological pathways in vulvar disorders; vulvar carcinoma (VC) and its precursors; vulvar squamous intraepithelial lesions (VIN).

**Background of the invention**

Vulvar carcinoma (VC) is a genital malignancy with age-standardized incidence rates ranging worldwide between 0.5 and 1.5 per 100,000. The current ASR in Poland is 1.13; 515 new cases of VC were diagnosed and 267 VC-related deaths were recorded in 2014 (age-standardized mortality rate 0.5) [Wojciechowska et al.]. It is widely recognized that vulvar squamous cell carcinoma (VSCC) develops on the basis of vulvar squamous intraepithelial lesions (VIN). In the classification adopted in 2015 by the International Society for the Study of Vulvar Disease (ISSVD), vulvar precancerous lesions are divided into two types, differing in their pathogenesis and clinical significance: high grade-squamous intraepithelial lesion (HSIL) previously classified as VIN usual type (uVIN) and differentiated vulvar intraepithelial neoplasia (dVIN) [Bornstein J et al.]. The risk of progression from uVIN to VSCC is low and ranges from 3 to 16%, while for dVIN it reaches 32,8%. In the United States, between 1973 and 2000, the incidence of VIN increased by 411% against 20% for invasive cancers [Akerman G et al.]. Vulvar squamous cell carcinoma (VSCC) is the most common histological type of VC, representing approximately 90 % of lesions. Five-year survival rate for VC patients is approximately 90% for early stages and 20% for advanced stages of the disease (http://seer.cancer.gov/statfacts/html/vulva.html).

Vulvar cancer diagnosis

Most VC and VIN are located in the labia majora, but other possible sites include the labia minora, clitoris, mons, or perineum. In patients with HPV-negative tumours, VC often presents as a single mass or ulcer on the labia majora or minora. In HPV-positive tumours, multifocal lesions and concurrent cervical neoplasia are more common.

There is no specific screening method to detect VC and its precursor lesions. Although many cases may be asymptomatic, pruritus and pain, irritation is a common symptom; vulvar bleeding or discharge may also occur. Most patients present with early-stage (i.e. localized) disease. If symptoms are suspicious for VC, the diagnosis must be primarily based on the clinical work-up. The following methods are used to identify precancerous lesions and carcinomas:

- clinical examination, consisting of careful inspection and palpation of the area including the inguinal region;

- vulvoscopy with the application of acetic acid.

All suspicious lesions must be individually biopsied and examined histologically as cancer may be seen in areas with multifocal vulvar intraepithelial neoplasia. The biopsy should be taken from the areas of the lesion that appear most abnormal. If multiple abnormal areas are present, then multiple biopsies should be taken to "map" all potential sites of vulvar pathology.

There are several diagnostic and differential diagnostic considerations that come into play in the pathological evaluation of VC in general, and its precursor lesions in particular. Thorough sampling of VIN is important, because 3.2-18.8% of biopsies can have unsuspected stromal invasion and be finally diagnosed as cancer. Also the interobserver variability amongst pathologists for assessing invasion is suboptimal ranging from 0.24 for diagnosing invasion and 0.51 for measuring depth of invasion. Also pathologic evaluation may not be possible for specimens taken from areas of extensive necrosis.

Xiuhua Yang et al: "miRNA expression profile of vulvar squamous cell carcinoma and identification of the oncogenic role of miR-590-5p", ONCOLOGY REPORTS, vol. 35, no. 1, 22 October 2015 (2015-10-22), pages 398-408, XP055463468, ISSN; 1021-335X, DOI: 10.3892/or.2015.4344 compared the miRNA expression profiles of VSCC and adjacent non-cancerous samples and investigated the mechanism of action of miR-590-5p in the A431 human VSCC cell line. The target of miR-590-5p was identified to elucidate the possible function of miR-590-5p in VSCC. No bodily fluids were examined in said document.

US 2009/075258 A1 discloses methods for normalizing miRNA quantification data in a biological sample. Said application described methods of quantifying a target miRNA in a biological sample by measuring the amount of a target miRNA and at least one reference oncomir in a reaction volume, and normalizing the amount of target miRNA to the amount of one or more reference oncomirs.

WO 2013/022786 A2 describes methods based on determining amounts of one or more exosome-derived micro-RNAs correlated with a lung cancer or a head and neck cancer in a biological sample from a subject.

Beatriz Maia et al: "MiR-223-5p works as an oncomiR in vulvar carcinoma by TP63 suppression", Oncotarget, 9 November 2014 (2014-11-09), XP055463216, DOI: 10.18632/oncotarget.10247 describes a role of miR-223-5p in a metastatic vulvar cancer cell line (SW962). Disclosed experiments conducted on solid vulvar carcinoma tissues and SW962 cells pointed to miR-223-5p overexpression as a putative pathological mechanism of vulvar cancer invasion and a promising therapeutic target.

No molecular tests nor markers allowing precise and non-invasive diagnosis and/or differentiation of malignant and premalignant lesions of the vulva have been developed to date.

To our knowledge, no one has, so far, conducted studies aiming at the identification of diagnostic or prognostic markers among the circulating microRNAs in VSCC and VIN. The identified microRNAs, demonstrating changes in blood levels likely related to prognosis, may provide basis for the development of a non-invasive test. Such test should allow diagnosis of VIN lesions and its differentiation from other benign conditions without malignant potential, early monitoring of the disease in patients with VIN, more precise VSCC staging, preoperative prognosis of lymph node status and ultimately serve as a prognostic and predictive marker for monitoring efficacy of therapy in VSCC patients.

The constantly increasing number of new VIN and VC cases and unsatisfactory treatment outcomes are a significant societal problem. Therefore, there is a great need to develop an effective, minimally invasive test that would allow to stratify patients with VIN and VSCC and to determine the risk of progression from VIN to VSCC.

## Summary of the invention

It has now surprisingly been found that a biological test (a biomarker) measured/assessed in the blood could considerably contribute to the improvement of treatment outcomes in VIN and VSCC, as such test could be used as a screening method allowing to identify VIN patients at the high-risk of disease progression to VSCC.

A combined signature consisting of microRNA determined in plasma serves to indicate which individuals among individuals with VIN are more likely to have VSCC and, at the same time, serves to rule out the presence of VSCC in the others.

The combined signature is based, inter alia, on the identification of specific miRNAs that have altered expression levels in the blood as well as on the identification of specific miRNAs with stable expression, i.e. normalizers (reference molecules).

The present invention provides a method of assessing whether a subject has, or is at risk of developing vulvar squamous cell carcinoma (VSCC) or vulvar squamous intraepithelial lesions (VIN) according to claim 1.

Furthermore the present invention provides a method of identifying one or more biomarkers for VIN or VSCC in a subject according to claim 10.

Embodiments of the invention are disclosed in the dependent claims.

A kit for performing the method of assessing whether a subject has or is at risk of developing VSCC or VIN is also disclosed herein but not claimed.

## Brief description of the drawing

Fig.1. Expression levels of four selected miRNAs in plasma samples of VIN and VSCC patients.

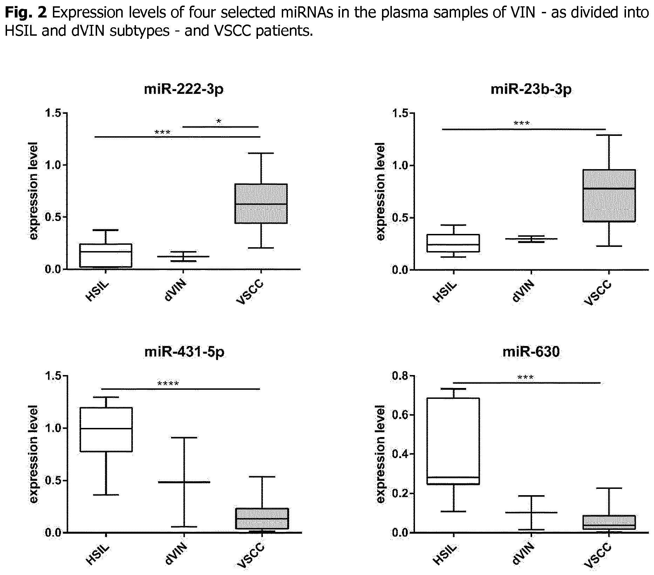

Fig. 2. Expression levels of four selected miRNAs in the plasma samples of VIN - as divided into HSIL and dVIN subtypes - and VSCC patients.

Fig. 3. Receiver operating characteristics (ROC) curve based on the normalized expression levels of miR-222-3p and miR-431-5p.

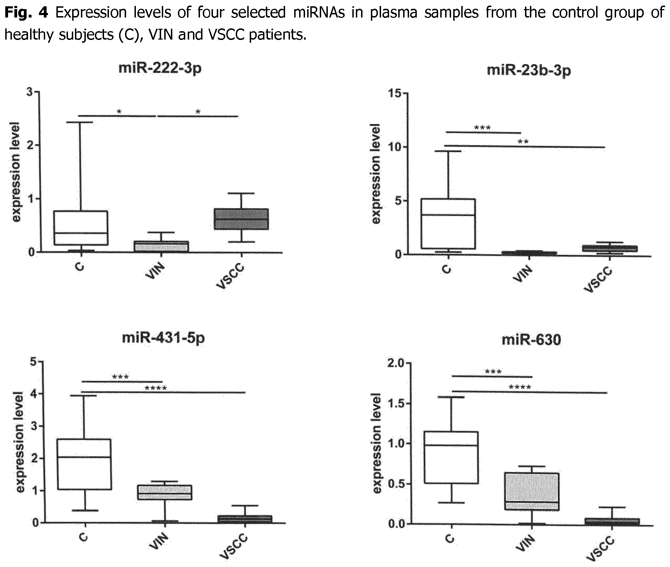

Fig. 4. Expression levels of four selected miRNAs in plasma samples of the control group of healthy subjects (C), as well as of VIN and VSCC patients.

## Definitions

The term "microRNA", "miRNA" or "miR" used herein means a non-coding RNA that regulates the translation of the coding mRNA(s) and is between 18 and 25-nucleotide long. These molecules are generally considered to be negative regulators of gene expression [Guo H et al.], yet they may also function as inducers of translation [Vasudevan S. et al.]. miRNAs are involved in a variety of both normal and pathological biological processes.

Examples and sequences of 1881 Homo sapiens miRNAs have been identified and are found in the miRNA database known as miRbase (http://www.mirbase.org/cgi-bin/mirna_summary.pl?org=hsa). MicroRNAs are named using the "miR" prefix and a unique identifying number (e.g., miR-1, miR-2, ... miR-89, etc.). The names/identifiers in the database are of the form such as hsa-mir-121. The first three letters signify the organism. The mature miRNA is designated miR-121 in the database and in much of the literature, whilst mir-121 refers to the miRNA gene and also to the predicted stem-loop portion of the primary transcript. Distinct precursor sequences and genomic loci that express identical mature sequences get names of the form such as hsa-mir-121-1 and hsa-mir-121-2. Lettered suffixes denote closely related mature sequences - for example hsa-miR-121a and hsa-miR-121b that would be expressed from precursors hsa-mir-121a and hsa-mir-121b, respectively. miRNA cloning studies sometimes identify two ~22nt sequences miRNAs which originate from the same predicted precursor. When the relative abundancies clearly indicate which is the predominantly expressed miRNA, the mature sequences are assigned names of the form miR-56 (the predominant product) and miR-56* (from the opposite arm of the precursor). When the data are not sufficient to determine which sequence is the predominant one, names like miR-142-5p (from the 5' arm) and miR-142-3p (from the 3' arm). Criteria and conventions for miRNA identification and naming are described by [V. Ambros et al. RNA 2003, 9(3):277-279].

The term "VSCC" provided herein refers to vulvar squamous cell carcinoma. VSCC refers to any primary or secondary non-neoplastic malignant condition affects squamous cell epithelia of the vulva. VSCC can be of any subtype, e.g., but not limited to warty, basaloid, keratinizing and non-keratinizing type. Variants of VSCC are classified based upon the microscopic findings, when examined by a pathologist under the microscope [WHO Classification of Tumours of Female Reproductive Organs WHO/IARC Classification of Tumours, 4th Edition, Volume 6, Year: 2014. Edited by Kurman RJ, Carcangiu ML, Herrington CS, Young RH. ISBN-13 978-92-832-2435-8].

The term "VIN" provided herein refers to vulvar intraepithelial neoplasia. VIN denotes a squamous intraepithelial lesion of the vulva that shows dysplasia with varying degrees of atypia. VIN comprises high-grade squamous intraepithelial lesion (HSIL, VIN usual type, uVIN), which is associated with developing into the warty and basaloid type carcinoma (which is associated with oncogenic HPV infection) and differentiated vulvar intraepithelial neoplasia, also known as dVIN, which is associated with vulvar dermatoses such as lichen sclerosus and also with atypia of the squamous epithelium. Therefore, the term VIN as used herein comprises both HSIL and dVIN lesions. The diagnosis of VIN is based on a careful inspection and a targeted biopsy of a visible vulvar lesion.

The term "healthy subject" provided herein refers to the subject which is diagnosed of not having VIN nor VSCC.

The terms "measuring the expression level", "measuring the level of miRNA" or "measuring the level of miRNA gene product" are used interchangeably in the art. These terms concern measuring a number of transcript copies produced in gene transcription process. The measuring, as used herein, refers to any method of detection, quantification, identification, visualisation, assessment, profiling, abundance measurement or analysis of miRNA level.

The expression level of miRNA can be measured using any technique that is suitable for detecting miRNA in a biological sample. Suitable techniques for determining miRNA expression level in cells from a biological sample are well known to those of skill in the art. In particular the following techniques may be applied: RT-PCR, quantitative RT-PCR (RT-qPCR), Northern blot analysis [Várallyay E, Burgyán J, Nat Protoc. 2008;3(2):190-6], hybridization to microarrays [Chang-Gong Liu CG at el. Nature Protocols 2008; 3: 563 - 578], miRNA sequencing, in particular next generation sequencing (NGS) [Tam S, de Borja R, Tsao MS and McPherson JD. Laboratory Investigation 2014, 94: 350-358], miRNA detection by microscopy with in situ hybridization (ISH) [Urbanek MA, Nawrocka AU and WJ. Int J Mol Sci. 2015; 16(6): 13259-13286], enzymatic luminescence miRNA assay [Sun Y, Gregory KJ, Chen NG, Golovlev V. Analytical Biochemistry 2012; 429(1):11-17].

The term "Ct" value, as used herein, relates to the value of threshold-cycle obtained in quantitative real-time PCR (qPCR) experiment. The normalized expression level is calculated by the $2^{-\Delta Ct}$ method, quantitating relative microRNA expression.

The term "normalizer", as used herein, relates to reference molecule, which should demonstrate stable expression level, storage stability, extraction, and quantification efficiency equivalent to that of the target of interest. Normalizers are used in order to minimize differences that could arise from sample procurement, stabilization, RNA extraction or quantification methods, or sample-to-sample inconsistencies.

The term "about", as used herein, refers to measurement uncertainty of the specified method and is used to reflect experimental deviations, which may be found for different measurements of the same sample.

An alteration in the level of the miRNA gene may be an increase or a decrease.

Functional variant is understood as a miRNA which varies by one nucleotide.

## Detailed description of the invention

In the first broad aspect, there is provided herein, a method of assessing whether a subject has, or is at risk of developing a vulvar-related disorder, in particular premalignant or malignant, determining prognosis of a subject with vulvar disorder comprising measuring at least one biomarker in a test sample.

In one embodiment, a method of assessing whether a subject has, or is at risk of developing vulvar squamous cell carcinoma (VSCC) or vulvar squamous intraepithelial lesions (VIN), comprises the steps of:

- measuring the expression level of at least one biomarker in a sample of the test subject, wherein the biomarkers are selected from a group consisting of miR-222-3p, miR-23b-3p, miR-431-5p and miR-630 or functional variants thereof,

- measuring the expression level of a normalizer in the sample of the test subject, wherein the combination of hsa-miR-93-5p and hsa-miR-425-5p is used as a normalizer,

- normalizing the measured expression level of at least one said biomarker against the normalizer to obtain a normalized value of the expression level of at least one said biomarker,

- comparing the normalized value of the expression level of at least one said biomarker with a normalized reference expression level,

- wherein the comparison of said normalized value of the expression level of at least one said biomarker to said normalized reference expression level is indicative to determine whether the subject has, or is at risk of developing vulvar squamous cell carcinoma or vulvar squamous intraepithelial lesions,

- and wherein the sample is whole blood; blood fraction such as plasma, serum, blood cell fractions or platelets.

The measured expression level is normalized against the combination of hsa-miR-93-5p and hsa-miR-425-5p and the average normalized value is calculated, or the expression level can be normalized against a combination of reference molecules.

Said reference expression level is one or more values established from one or more biological samples. The samples may be samples obtained from healthy subjects or subjects having or being at risk of developing VSCC or VIN. Reference expression levels may be expressed as reference intervals (RI) [Jones G, Barker A. Clin Biochem Rev. 2008; 29 (Suppl 1): S93-S97]. The current paradigm for pathology reference intervals is for each laboratory to determine its own interval for use with each test offered by the laboratory [ Jones GR, Barker A, Tate J, Lim CF and Robertson K. Clin Biochem Rev. 2004; 25(2): 99-104].

In certain embodiments, the sample comprises one or more of serum or blood plasma or whole blood samples. In preferred embodiment, the sample is blood plasma.

In certain embodiments, at least one biomarker is differentially expressed in the plasma of the healthy subject (C), subject with tumour (VSCC) and/or preinvasive lesion (VIN), and is one or more of miR-222-3p, miR-23b-3p, miR-431-5p and miR-630, or variants thereof.

In one embodiment the normalized value for miR-222-3p expression level in a range about 0.26 - 1.0 indicates VSCC or healthy subject, and/or the normalized value for miR-222-3p expression level in a range about 0.0 - 0.22 indicates VIN. Preferably the normalized value for miR-222-3p expression level being about 0.0, 0.1 or 0.2 indicates VIN.

In one embodiment the normalized value for miR-431-5p expression level in a range about 0.0 - 0.4 indicates VSCC, and/or the normalized value for miR-431-5p expression level in a range about 0.5 - 1.3 indicates VIN, and/or the normalized value for miR-431-5p expression level in a range about 1.4 - 2.6 indicates healthy subject. Preferably the normalized value for miR-431-5p expression being about 0.0, 0.1, 0.2, 0.3 or 0.4 indicates VSCC. Preferably the normalized value for miR-431-5p expression level being about 0.5, 0.6, 0.7, 0.8, 0.9, 1.0, 1.1, 1.2 or 1.3 indicates VIN. Preferably the normalized value for miR-431-5p expression level larger than about 1.4 indicates healthy subject.

In one embodiment the normalized value for miR-630 expression level in a range about 0.0 - 0.15 indicates VSCC, and/or the normalized value for miR-630 expression level in a range about 0.16 - 0.6 indicates VIN, and/or the normalized value for miR-630 expression level in a range about 0.7 - 1.1 indicates healthy subject. Preferably the normalized value for miR-630 expression level being about 0.0, 0.1 or 0.15 indicates VSCC. Preferably the normalized value for miR-630 expression level being about 0.16, 0.2, 0.3, 0.4, 0.5 or 0.6 indicates VIN. Preferably the normalized value for miR-630 expression level larger than about 0.7 indicates healthy subject.

In one embodiment the normalized value for miR-23b-3p expression level in a range about 0.35 - 1.3 indicates VSCC, and/or the normalized value for miR-23b-3p expression level in a range about 0.1 - 0.34 indicates VIN, and/or the normalized value for miR-23b-3p expression level in a range about 1.8 - 4.8 indicates healthy subject. Preferably the normalized value for miR-23b-3p expression level being about 0.35, 0.4, 0.5, 0.6, 0.7, 0.8, 0.9, 1.0., 1.1, 1.2 or 1.3 indicates VSCC. Preferably the normalized value for miR-23b-3p expression level being about 0.1, 0.2, 0.3 or 0.34 indicates VIN. Preferably the normalized value for miR-23b-3p expression level being larger than 1.8 indicates healthy subject.

In certain embodiments, the level of the biomarker in the blood sample of VSCC patient is greater or lower than the level of the corresponding biomarker in the blood sample of VIN patient.

In preferred embodiment, RT-qPCR is used for measuring the expression level of at least one biomarker selected from one or more of miR-222-3p, miR-23b-3p, miR-431-5p and miR-630, or functional variants thereof.

In one embodiment, the invention provides a method of assessing whether a subject has, or is at risk of developing a VIN or VSCC by combined use of the miR-222-3p and miR-431-5p biomarkers.

In another aspect, the invention provides a method of identifying one or more biomarkers for VIN or VSCC in a subject, comprising the steps of:

- measuring the expression level of at least one candidate biomarker in a sample of the test subject,
- measuring the expression level of at least one normalizer in the sample of the test subject, wherein the combination of hsa-miR-93-5p and hsa-miR-425-5p is used as a normalizer,
- normalizing the measured expression level of at least one said biomarker against normalizer to obtain the normalized value of the expression level of at least one said biomarker,
- comparing the normalized expression level with normalized reference expression level, wherein the comparison of said normalized expression level of at least one said biomarker to said normalized reference expression level is indicative to determine whether the candidate biomarker is a biomarker for VIN or VSCC,
- wherein said reference expression level is one or more values established from one or more biological samples,
- and wherein the sample is whole blood; blood fraction such as plasma, serum, blood cell fractions or platelets; preferably blood fraction, more preferably blood plasma.

To better understand the invention there is disclosed herein a method of screening for one or more biomarkers for VSCC or VIN in a subject, comprising: obtaining a sample of blood plasma from a subject, measuring the expression level of at least one biomarker, wherein the biomarkers are selected from one or more of miR-222-3p, miR-23b-3p, miR-431-5p and miR-630, or functional variants thereof. The expression level of at least one said biomarker is preferably normalized against normalizer present in the sample to obtain the normalized value of the expression level of at least one said biomarker and the normalized value of the expression level is used for further analysis. The normalizers are preferably one or more molecules selected from hsa-miR-93-5p, hsa-miR-425-5p, hsa-miR-191-5p or a combination thereof, most preferably the combination of hsa-miR-93-5p and hsa-miR-425-5p.

Furthermore, there is disclosed herein a method of screening for one or more biomarkers for VSCC or VIN in a subject, comprising: obtaining a sample of blood plasma from a subject, conducting reverse transcription quantitative real-time polymerase chain reaction (RT-qPCR), and quantifying one or more than one biomarkers differentially expressed between blood of VSCC patients, VIN patients and healthy patients, wherein the biomarkers are selected from one or more of miR-222-3p, miR-23b-3p, miR-431-5p and miR-630, or functional variants thereof. The expression level of at least one said biomarker is preferably normalized against normalizer present in the sample to obtain the normalized value of the expression level of at least one said biomarker, and the normalized value of the expression level is used for further analysis. The normalizers are preferably one or more molecules selected from hsa-miR-93-5p, hsa-miR-425-5p, hsa-miR-191-5p or a combination thereof, most preferably the combination of hsa-miR-93-5p and hsa-miR-425-5p.

Measuring the expression level of at least one biomarker selected from one or more of miR-222-3p, miR-23b-3p, miR-431-5p and miR-630, or functional variants thereof may be performed using a method comprising northern blotting, hybridization to microarrays, microRNA sequencing (e.g., next generation sequencing), single cell miRNA detection by microscopy with in situ hybridization (ISH), enzymatic luminescence miRNA assay. Furthermore, other methods of microRNA detection known in the art can be used for measuring the expression level of at least one said biomarker.

There is disclosed herein, a method for influencing transcript abundance and/or protein expression of target miR in the blood of the subject in need thereof, comprising deregulating one or more microRNAs selected from one or more of miR-222-3p, miR-23b-3p, miR-431-5p and miR-630, or functional variants thereof. The method includes altering expression of one or more of miR-222-3p, miR-23b-3p, miR-431-5p and miR-630 to inhibit the protein expression of cancer-related genes.

There is further disclosed herein, a method for screening for vulvar pathology in a subject in need thereof and a method for follow-up after treatment for vulvar pathology in a subject in need thereof.

There is further disclosed herein, a blood sample microRNA signature for a vulvar related pathology comprising: one or more of: miR-222-3p, miR-23b-3p, miR-431-5p and miR-630, or a functional variants or combinations thereof that are up-regulated or that are down-regulated.

There is further disclosed herein, a method for regulating one or more of genes expressed by VSCC or VIN cells, comprising the step of altering expression of miR-222-3p, miR-23b-3p, miR-431-5p and miR-630 in vulvar cells.

There is further disclosed herein, use of mimics of miR-431-5p and miR-630, or functional variants or combinations thereof, as a microRNAs targeting at least one gene in vulvar cells and thus inhibiting protein expression of such gene.

There is further disclosed herein, use of inhibitors of miR-222-3p and miR-23b-3p, or functional variants or combinations thereof, as a microRNAs targeting at least one gene in vulvar cells and thus increasing protein expression of such gene.

There is further disclosed herein, a method of treating vulvar disorders in a subject who has vulvar disorder in which at least one biomarker is down-regulated or up-regulated in the plasma, serum or whole blood of the subject with vulvar disorder, administrating to the subject an effective amount of mimic or inhibitor of at least one biomarker, or a variant or biologically-active fragment thereof, such that proliferation of VSCC or VIN cells in the subject is inhibited, wherein the biomarker is selected from one or more of miR-222-3p, miR-23b-3p, miR-431-5p and miR-630, or functional variants thereof.

There is further disclosed herein, a pharmaceutical composition for treating vulvar disorders, comprising at least one isolated biomarker, and a pharmaceutically-acceptable carrier, wherein the biomarker is selected from one or more of miR-222-3p, miR-23b-3p, miR-431-5p and miR-630, or functional variants thereof.

To better illustrate the invention there is also disclosed a kit for performing a method of assessing whether a subject has, or is at risk of developing VSCC or VIN by quantitative RT-PCR, comprising primers and probes specific to miRNA sequences of at least one of miR-222-3p, miR-23b-3p, miR-431-5p and miR-630. The term "target nucleotide sequence" or "target nucleotide" as used herein, refers to the polynucleotide sequence that is sought to be detected and/or quantified, i.e. the sequence that is targeted by the microRNA-specific primers and/or probes. The target miRNA nucleotide sequences are amplified and detected, thereby simultaneously detecting the expression levels of miR-222-3p, miR-23b-3p, miR-431-5p and miR-630. Alternatively, the methods may be applied directly on cDNA without the need for reverse transcription.

The disclosed kit typically comprises specific primers and probes specific to miRNA sequences of at least one of miR-222-3p, miR-23b-3p, miR-431-5p and miR-630, dNTP, as well as polymerase, reaction buffer and $Mg^{2+}$.

**Examples**

**Identification of reference microRNA**

The blood samples obtained from 44 patients with VIN and VSCC were included in the study. Furthermore blood samples obtained from 14 healthy subjects were used as a control group C. Among seventeen patients treated for VIN between December 2013 and October 2014 enrolled 12 and 5 were diagnosed with HSIL and d-VIN, respectively. The median age of patients treated for VIN was 58,4 years (range 33-79). Overall, twenty seven patients surgically treated for primary VSCC (T1-2, N0-2, M0) at the Maria Sklodowska-Curie Institute - Oncology Center in Warsaw (CO-I) between February 2005 and May 2011 were recruited. No patient received preoperative chemotherapy or radiotherapy. The median age of patients treated for VSCC was 75.4 years (range 54-92). The study was approved by the Independent Ethics Committee of all the institutions, CO-I (No. 44/2002, 16/2015), HCC (No. 15/2014) and WUM (No. 247/2015), and all patients gave their informed consent.

Plasma samples were obtained from patients' blood before their surgical treatment and stored at -70°C until RNA isolation. Total RNA was isolated from 200 µl of plasma using miRNeasy Mini Kit (Qiagen) and miRNA was reverse transcribed using miScript II RT kit (Qiagen) according to the manufacturer's protocol.

Expression levels of six candidate reference miRNAs, i.e. hsa-miR-93-5p, hsa-miR-425-5p, hsa-miR-16-5p, hsa-miR- 103a-3p, hsa-miR-191-5p, hsa-miR-423-5p, were analysed by quantitative PCR (RT-qPCR) using the miScript miRNA Arrays (Qiagen) according to the manufacturer's protocol. The microRNA molecules in question served as candidate normalizers. Reactions were performed in 7500 Fast Real-Time PCR System (Applied Biosystems) in a final volume of 25 µl reaction mix, according to the arrays' manufacturer instructions (Qiagen).

Gene expression data were analysed using geNorm™ algorithm [https://genorm.cmgg.be/] integrated into DataAssist™ Software (Applied Biosystems, Thermo Fisher Scientific, Carlsbad, CA, USA). RefFinder, a web-based application which integrates three most commonly used normalization algorithms (geNorm, Normfinder and BestKeeper), was used for results cross-validation [Xie et al.].The algorithm used allowing to determine the most stable normalizers from a set of tested candidate normalizers in a given sample panel. It calculates a gene stability measure (M) according to formulas described by Vandesompele et al. Genes with the lowest M values (<1.5) have the most stable expression.

Our analysis of miRNA expression data with the algorithm identified hsa-miR-93-5p (M= 1.3276) as the gene that retained the greatest robustness in all the plasma samples analysed, i.e. of both VIN and VSCC patients (Tab. 1, bolded font). We identified hsa-miR-93-5p (M=1. 4481) as the most stable reference miRNA (normalizer) for VIN. In plasma samples of patients with VSCC hsa-miR-425-5p (M=1.017) followed by hsa-miR-93-5p (M=1.044) and hsa-miR-191-5p (M=1.3937) were the most stable reference miRNA (normalizer).

This finding enabled us to avoid the common error of using inadequate normalizers in qPCR analysis of microRNA expression, such as snoRNAs which have variable expression due to their involvement in carcinogenesis [Mannoor et al.].

**Tab.1.** Putative reference miRNAs (normalizers) and their expression stability values in plasma samples of VIN and VSCC patients.

| miRNA | miRBase accession number | Stability values for candidate reference genes in plasma calculated by the geNorm™ program | | |
|---|---|---|---|---|
| | | VIN | VSCC | VSCC and VIN |
| hsa-miR-93-5p | MIMAT0000093 | **1.4481** | 1.044 | **1.3276** |
| hsa-miR-425-5p | MIMAT0003393 | 1.6850 | **1.017** | 1.3909 |
| hsa-miR-16-5p | MIMAT0000069 | 1.6182 | 1.4196 | 1.6871 |
| hsa-miR-103a-3p | MIMAT0000101 | 2.1591 | 1,4511 | 1.8671 |
| hsa-miR-191-5p | MIMAT0000440 | 2.5022 | 1.3937 | 2.0446 |
| hsa-miR-423-5p | MIMAT0004748 | 1.8651 | 1.4748 | 1.6876 |

**Identification of biomarker microRNAs**

Next, we evaluated expression level of 20 microRNAs in the plasma samples of 23 patients: 11 with VIN (9 diagnosed with HSIL and 2 with d-VIN) and 12 with VSCC. Briefly, total RNA was isolated from 200 $\mu$l of plasma using miRNeasy Mini Kit (Qiagen) and miRNA was reverse transcribed using miScript II RT kit (Qiagen) according to the manufacturer's protocol. Expression levels of 20 microRNAs listed in Tab. 2, were analysed by quantitative RT-PCR (RT-qPCR) using the miScript miRNA Arrays (Qiagen) according to the manufacturer's protocol. To normalize the results we used the combination of the most stable reference microRNA (normalizer) i.e. hsa-miR-93-5p (miRBase Accession No. MIMAT0000093, having mature sequence CAAAGUGCUGUUCGUGCAGGUAG) and hsa-miR-425-5p (miRBase Accession No. MIMAT0003393, having mature sequence AAUGACACGAUCACUCCCGUUGA) (Table 1, bolded font). Reactions were performed in 7500 Fast Real-Time PCR System (Applied Biosystems) in a final volume of 25 $\mu$l reaction mix, according to the arrays' manufacturer instructions (Qiagen). The collected data were analysed using threshold-cycle (Ct) values for the miRNAs with the SDS 2.1 software (Applied Biosystems). The relative amounts of each miR in the plasma of patients with VIN and VSCC were calculated by the $2^{-\Delta Ct}$ method using DataAssist™ Software (Applied Biosystems). Expression levels for the selected miRs were graphed using GraphPad Prism version 6.07.

**Tab.2.** List of microRNAs surveyed in the plasma samples of VIN and VSCC patients.

| Mature miRNA ID | miRBase Accession No. | Mature sequence |
|---|---|---|
| hsa-miR-630 | MIMAT0003299 | AGUAUUCUGUACCAGGGAAGGU |
| hsa-miR-19b | MIMAT0000074 | UGUGCAAAUCCAUGCAAAACUGA |
| hsa-miR-130b | MIMAT0000691 | CAGUGCAAUGAUGAAAGGGCAU |
| hsa-miR-21 | MIMAT0000076 | UAGCUUAUCAGACUGAUGUUGA |
| hsa-miR-532-5p | MIMAT0002888 | CAUGCCUUGAGUGUAGGACCGU |
| hsa-miR-210 | MIMAT0026475 | AGCCCCUGCCCACCGCACACUG |
| hsa-miR-20a | MIMAT0000075 | UAAAGUGCUUAUAGUGCAGGUAG |
| hsa-miR-222-3p | MIMAT0000279 | AGCUACAUCUGGCUACUGGGU |
| hsa-miR-144 | MIMAT0000436 | UACAGUAUAGAUGAUGUACU |
| hsa-miR-155 | MIMAT0000646 | UUAAUGCUAAUCGUGAUAGGGGU |

(continued)

| Mature miRNA ID | miRBase Accession No. | Mature sequence |
|---|---|---|
| hsa-miR-133a | MIMAT0026478 | AGCUGGUAAAAUGGAACCAAAU |
| hsa-miR-1 | MIMAT0000416 | UGGAAUGUAAAGAAGUAUGUAU |
| hsa-miR-206 | MIMAT0000462 | UGGAAUGUAAGGAAGUGUGUGG |
| hsa-miR-29b | MIMAT0000100 | UAGCACCAUUUGAAAUCAGUGUU |
| hsa-miR-145* | MIMAT0004601 | GGAUUCCUGGAAAUACUGUUCU |
| hsa-miR-7 | MIMAT0000252 | UGGAAGACUAGUGAUUUUGUUGU |
| miR-23b-3p | MIMAT0000418 | AUCACAUUGCCAGGGAUUACC |
| miR-431-5p | MIMAT0001625 | UGUCUUGCAGGCCGUCAUGCA |
| miR-543 | MIMAT0004954 | AAACAUUCGCGGUGCACUUCUU |
| let-7c | MIMAT0000064 | UGAGGUAGUAGGUUGUAUGGUU |

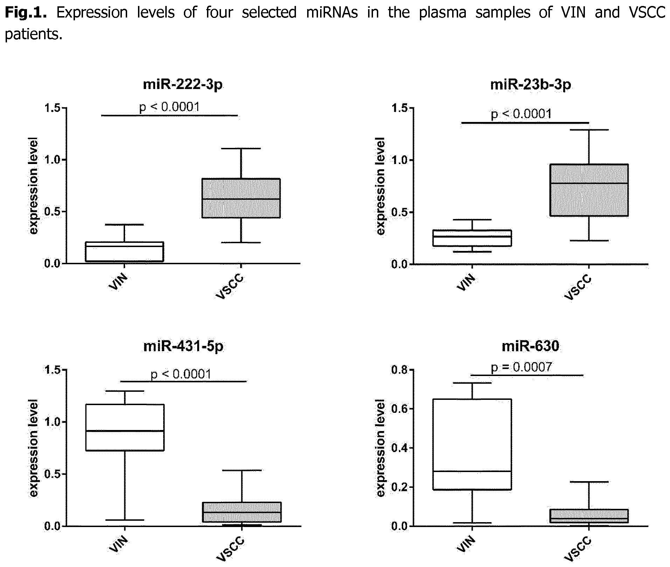

The results pointing at four microRNA molecules which levels were differentiating the tested samples (VSCC *vs* VIN) are depicted in Fig. 1.

The results identify four microRNA molecules that differentiated tested samples (VSCC *vs* VIN) and these differences were statistically significant. The current disclosure reveals that expression level of miR-222-3p, miR-23b-3p, miR-431-5p and miR-630 are circulating biomarkers for differential diagnosis of VIN and VSCC. The levels of miR-222-3p and miR-23b-3p were decreased in the blood of patients with VIN and increased in VSCC, whereas the levels of miR-431-5p and miR-630 were increased in the blood of patients with VIN and decreased in VSCC.

Furthermore, the results obtained for a control group show that miR-23b-3p, miR-431-5p and miR-630 are circulating biomarkers suitable for assessing whether a subject has, or is at risk of developing VIN or VSCC.

The method of comparison of expression levels of miR-222-3p, miR-23b-3p, miR-431-5p and miR-630 is of high prediction value and is indicative for diagnosis of vulvar squamous cell carcinoma or vulvar squamous intraepithelial lesions, which was confirmed by ROC (receiver operating characteristics). AUCs (areas under the curve) for ROCs were as follows:

- 0.970 for miR-222-3p,
- 0.939 for miR-23b-3p,
- 0.924 for miR-431-5p and
- 0.894 for miR-630.

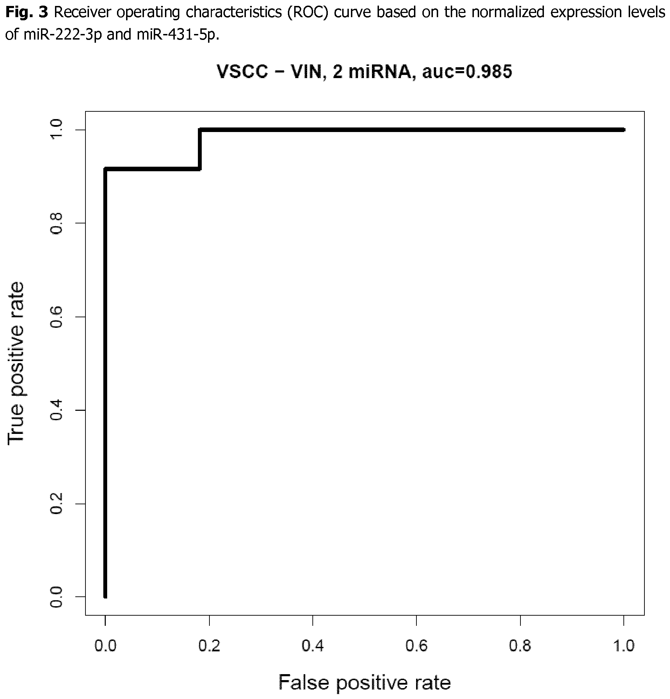

As an example, the normalized expression levels miR-222-3p and miR-431-5p were used simultaneously to estimate the sensitivity and specificity of the sample classifier (Fig. 3). This combination classifier had an AUC of 0.985 and accurately identified patients with VIN and VSCC, thus exhibited excellent performance.

Moreover, our analysis revealed the possibility of building a sample type predictor according to the following formula:

**Predictor Score = (coeff x inverse logit of X) + (coeff x inverse logit of Y)**

whereas

coeff is -1.836
X is the value of normalized expression of miR-222-3p
Y is the value of normalized expression of miR-431-5p

The resulting score > 0.5 assigns the sample to the VSCC group, while the score < 0.5 assigns the sample to the VIN group.

Non-patent literature:

1. Wojciechowska U, Didkowska J. Zachorowania i zgony na nowotwory zzłośliwe w Polsce. Krajowy Rejestr Nowotworów, Centrum Onkologii - Instytut im. Marii Sktodowskiej - Curie

2. Bornstein J, Bogliatto F, Haefner HK, Stockdale CK, Preti M, Bohl TG, Reutter J, Committee IT: The 2015 international society for the study of vulvovaginal disease (issvd) terminology of vulvar squamous intraepithelial lesions. J Low Genit Tract Dis 2016;20:11-14.

3. Ji T, Zheng ZG, Wang FM, Xu U, Li LF, Cheng QH, Guo JF, Ding XF: Differential microrna expression by solexa sequencing in the sera of ovarian cancer patients. Asian Pac J Cancer Prev 2014;15:1739-1743.Vasudevan S. et al.

4. V. Ambros et al. "A uniform system for microRNA annotation". RNA 2003 9(3):277-279.

5. WHO Classification of Tumours of Female Reproductive Organs WHO/IARC Classification of Tumours, 4th Edition, Volume 6, Year: 2014. Edited by Kurman RJ, Carcangiu ML, Herrington CS, Young RH. ISBN-13 978-92-832-2435-8

6. Várallyay E, Burgyán J, Havelda Z. MicroRNA detection by northern blotting using locked nucleic acid probes. Nat Protoc. 2008;3(2):190-6.

7. Chang-Gong Liu, George Adrian Calin, Stefano Volinia & Carlo M Croce MicroRNA expression profiling using microarrays Nature Protocols 3, 563 - 578 (2008)

8. Shirley Tam, Richard de Borja, Ming-Sound Tsao and John D McPherson, Robust global microRNA expression profiling using next-generation sequencing technologies, Laboratory Investigation (2014) 94, 350-358

9. Martyna O. Urbanek, Anna U. Nawrocka,and Wlodzimierz J. Krzyzosiak Small RNA Detection by in Situ Hybridization Methods Int J Mol Sci. 2015 Jun; 16(6): 13259-13286

10. YeSunKalvin J. GregoryNelson G. ChenValGolovlev Rapid and direct microRNA quantification by an enzymatic luminescence assay Analytical Biochemistry Volume 429, Issue 1, 1 October 2012, Pages 11-17

11. Graham Jones, Antony Barker Reference Intervals Clin Biochem Rev. 2008 Aug; 29 (Suppl 1): S93-S97.

12. Graham RD Jones, Antony Barker, Jill Tate, Chen-Fee Lim and Ken Robertson The Case for Common Reference Intervals Clin Biochem Rev. 2004 May; 25(2): 99-104.

13. Xie F, Xiao P, Chen D, et al. miRDeepFinder: a miRNA analysis tool for deep sequencing of plant small RNAs. Plant Mol Biol. Epub ahead of print 31 January 2012. DOI: 10.1007/s11103-012-9885-2

14. Vandesompele J, De Preter K, Patlyn F, et al. Accurate normalization of real-time quantitative RT-PCR data by geometric averaging of multiple internal control genes. Genome Biol 2002; 3: RESEARCH0034.

15. Mannoor K, Liao J, Jiang F.Small nucleolar RNAs in cancer. Biochim Biophys Acta. 2012;1826(1): 121-8

## Claims

1. A method of assessing whether a subject has, or is at risk of developing vulvar squamous cell carcinoma (VSCC) or vulvar squamous intraepithelial lesions (VIN), comprising the steps of:

   - measuring the expression level of at least one biomarker in a sample of the test subject, wherein the biomarkers are selected from a group consisting of miR-222-3p, miR-23b-3p, miR-431-5p and miR-630 or functional variants thereof,
   - measuring the expression level of a normalizer in the sample of the test subject, wherein the combination of hsa-miR-93-5p and hsa-miR-425-5p is used as a normalizer,
- normalizing the measured expression level of at least one said biomarker against the normalizer to obtain a normalized value of the expression level of at least one said biomarker,
- comparing the normalized value of the expression level of at least one said biomarker with a normalized reference expression level,
- wherein the comparison of said normalized value of the expression level of at least one said biomarker to said normalized reference expression level is indicative to determine whether the subject has, or is at risk of developing vulvar squamous cell carcinoma or vulvar squamous intraepithelial lesions,
- and wherein the sample is whole blood; blood fraction such as plasma, serum, blood cell fractions or platelets.

2. The method according to claim 1, wherein the sample is blood fraction, preferably blood plasma.

3. The method according to claim 1 or 2, wherein the comparison of said normalized value of the expression level of at least one said biomarker to said normalized reference expression level is represented by normalized expression level values indicative for diagnosis of VIN or VSCC.

4. The method according to claim 3, wherein the normalized value for miR-222-3p expression level in a range 0.0 - 0.22 indicates VIN.

5. The method according to claim 3, wherein the normalized value for miR-431-5p expression level in a range 0.0 - 0.4 indicates VSCC, and/or the normalized value for miR-431-5p expression level in a range 0.5 - 1.3 indicates VIN.

6. The method according to claim 3, wherein the normalized value for miR-630 expression level in a range 0.0 - 0.15 indicates VSCC, and/or the normalized value for miR-630 expression level in a range 0.16 - 0.6 indicates VIN.

7. The method according to claim 3, wherein the normalized value for miR-23b-3p expression level in a range 0.35 - 1.3 indicates VSCC, and/or the normalized value for miR-23b-3p expression level in a range 0.1 - 0.34 indicates VIN.

8. The method according to claim 3, wherein the normalized value for miR-23b-3p expression level in a range 1.8 - 4.8 indicates healthy subject, and/or the normalized value for miR-431-5p expression level in a range 1.4 - 2.6 indicates healthy subject, and/or the normalized value for miR-630 expression level in a range 0.7 - 1.1 indicates healthy subject.

9. The method according to any of the previous claims, wherein the selected biomarkers are miR-222-3p and miR-431-5p and are used together.

10. A method of identifying one or more biomarkers for VIN or VSCC in a subject, comprising the steps of:

- measuring the expression level of at least one candidate biomarker in a sample of the test subject,
- measuring the expression level of a normalizer in the sample of the test subject, wherein the combination of hsa-miR-93-5p and hsa-miR-425-5p is used as a normalizer,
- normalizing the measured expression level of at least one said biomarker against the normalizer to obtain the normalized value of the expression level of at least one said biomarker,
- comparing the normalized expression level with a normalized reference expression level, wherein the comparison of said normalized expression level of at least one said biomarker to said normalized reference expression level is indicative to determine whether the candidate biomarker is a biomarker for VIN or VSCC,
- wherein said reference expression level is one or more values established from one or more biological samples,
- and wherein the sample is whole blood; blood fraction such as plasma, serum, blood cell fractions or platelets; preferably blood fraction, more preferably blood plasma.

## Patentansprüche

1. Verfahren zur Beurteilung, ob ein Subjekt ein Plattenepithelkarzinom der Vulva (VSCC) oder eine intraepitheliale Plattenepithelläsion der Vulva (VIN) hat oder ob ein Risiko dafür besteht, umfassend die Schritte von

- Messen des Expressionsniveaus mindestens eines Biomarkers in einer Probe des Testsubjekts, wobei die Biomarker aus einer Gruppe ausgewählt sind, die aus miR-222-3p, miR-23b-3p, miR-431-5p und miR-630 oder funktionellen Varianten davon besteht,
- Messen des Expressionsniveaus eines Normalisierers in der Probe des Testsubjekts, wobei die Kombination aus hsa-miR-93-5p und hsa-miR-425-5p als Normalisierer verwendet wird,
- Normalisieren des gemessenen Expressionsniveaus von mindestens einem der Biomarker mit dem Normalisierer, um einen normalisierten Wert des Expressionsniveaus von mindestens einem der Biomarker zu erhalten,
- Vergleichen des normalisierten Werts des Expressionsniveaus von mindestens einem der Biomarker mit einem normalisierten Referenzexpressionsniveau,
- wobei der Vergleich des normalisierten Werts des Expressionsniveaus von mindestens einem der Biomarker mit dem normalisierten Referenzexpressionsniveau indikativ ist, um festzustellen, ob das Subjekt ein Plattenepithelkarzinom der Vulva oder eine intraepitheliale Plattenepithelläsion der Vulva hat oder ob ein Risiko dafür besteht,
- und wobei die Probe Vollblut, eine Blutfraktion wie Plasma, Serum, Blutzellfraktionen oder Blutplättchen ist.

2. Verfahren nach Anspruch 1, wobei die Probe eine Blutfraktion, vorzugsweise Blutplasma, ist.

3. Verfahren nach Anspruch 1 oder 2, wobei der Vergleich des normalisierten Werts des Expressionsniveaus von mindestens einem der Biomarker mit dem normalisierten Referenzexpressionsniveau durch normalisierte Expressionsniveauwerte dargestellt wird, die für eine Diagnose von VIN oder VSCC indikativ sind.

4. Verfahren nach Anspruch 3, wobei der normalisierte Wert für das miR-222-3p-Expressionsniveau in einem Bereich von 0,0-0,22 VIN anzeigt.

5. Verfahren nach Anspruch 3, wobei der normalisierte Wert für das miR-431-5p-Expressionsniveau in einem Bereich von 0,0-0,4 das VSCC anzeigt, und/oder der normalisierte Wert für das miR-431-5p-Expressionsniveau in einem Bereich von 0,5-1,3 VIN anzeigt.

6. Verfahren nach Anspruch 3, wobei der normalisierte Wert für das miR-630-Expressionsniveau in einem Bereich von 0,0-0,15 das VSCC anzeigt und/oder der normalisierte Wert für das miR-630-Expressionsniveau in einem Bereich von 0,16-0,6 VIN anzeigt.

7. Verfahren nach Anspruch 3, wobei der normalisierte Wert für das miR-23b-3p-Expressionsniveau in einem Bereich von 0,35-1,3 das VSCC anzeigt, und/oder der normalisierte Wert für das miR-23b-3p-Expressionsniveau in einem Bereich von 0,1-0,34 VIN anzeigt.

8. Verfahren nach Anspruch 3, wobei der normalisierte Wert für das miR-23b-3p-Expressionsniveau in einem Bereich von 1,8-4,8 ein gesundes Subjekt anzeigt und/oder der normalisierte Wert für das miR431-5p-Expressionsniveau in einem Bereich von 1,4-2,6 ein gesundes Subjekt anzeigt, und/oder der normalisierte Wert für das miR-630-Expressionsniveau in einem Bereich von 0,7-1,1 ein gesundes Subjekt anzeigt.

9. Verfahren nach einem der vorhergehenden Ansprüche, wobei die ausgewählten Biomarker miR-222-3p und miR-431-5p sind und zusammen verwendet werden.

10. Verfahren zum Identifizieren eines oder mehrerer Biomarker für VIN oder VSCC bei einem Subjekt, umfassend die Schritte von:

- Messen des Expressionsniveaus mindestens eines Kandidaten-Biomarkers in einer Probe des Testsubjekts,
- Messen des Expressionsniveaus eines Normalisierers in der Probe des Testsubjekts, wobei die Kombination aus hsa-miR-93-5p und hsa-miR-425-5p als Normalisierer verwendet wird,
- Normalisieren des gemessenen Expressionsniveaus von mindestens einem der Biomarker mit dem Normalisierer, um den normalisierten Wert des Expressionsniveaus von mindestens einem der Biomarker zu erhalten,
- Vergleichen des normalisierten Expressionsniveaus mit einem normalisierten Referenzexpressionsniveau,
- wobei der Vergleich des normalisierten Expressionsniveaus von mindestens einem der Biomarker mit dem normalisierten Referenzexpressionsniveau indikativ ist, um festzustellen, ob der Kandidaten-Biomarker ein Biomarker für VIN oder VSCC ist,
- wobei das Referenzexpressionsniveau ein oder mehrere Werte ist, die aus einer oder mehreren biologischen Proben ermittelt wurden,
- und wobei die Probe Vollblut, eine Blutfraktion wie Plasma, Serum, Blutzellfraktionen oder Blutplättchen ist; vorzugsweise Blutfraktion, bevorzugt Blutplasma.

**Revendications**

1. Procédé pour évaluer si un sujet a, ou risque de développer, un carcinome squameux vulvaire (VSCC) ou des lésions intraépithéliales squameuses vulvaires (VIN), comprenant les étapes consistant à :

   - mesurer le niveau d'expression d'au moins un biomarqueur dans un échantillon du sujet testé, les biomarqueurs étant sélectionnés dans un groupe constitué de miR-222-3p, miR-23b-3p, miR-431-5p et miR-630 ou de variantes fonctionnelles de ceux-ci,
   - mesurer le niveau d'expression d'un normalisateur dans l'échantillon du sujet testé, la combinaison de hsa-miR-93-5p et hsa-miR-425-5p étant utilisée comme normalisateur,
   - normaliser le niveau d'expression mesuré d'au moins un dit biomarqueur par rapport au normalisateur pour obtenir une valeur normalisée du niveau d'expression d'au moins un dit biomarqueur,
   - comparer la valeur normalisée du niveau d'expression d'au moins un dit biomarqueur avec un niveau d'expression de référence normalisé,

   dans lequel la comparaison de ladite valeur normalisée du niveau d'expression d'au moins un dit biomarqueur avec ledit niveau d'expression de référence normalisé est indicative pour déterminer si le sujet a, ou risque de développer, un carcinome squameux vulvaire ou des lésions intraépithéliales squameuses vulvaires, et dans lequel l'échantillon est du sang total ; une fraction sanguine telle que le plasma, le sérum, les fractions de cellules sanguines ou les plaquettes sanguines.

2. Procédé selon la revendication 1, dans lequel l'échantillon est une fraction sanguine, de préférence du plasma sanguin.

3. Procédé selon la revendication 1 ou 2, dans lequel la comparaison de ladite valeur normalisée du niveau d'expression d'au moins un dit biomarqueur avec ledit niveau d'expression de référence normalisé est représentée par des valeurs normalisées du niveau d'expression indiquant le diagnostic de VIN ou VSCC.

4. Procédé selon la revendication 3, dans lequel la valeur normalisée pour le niveau d'expression de miR-222-3p dans une plage de 0,0 à 0,22 indique VIN.

5. Procédé selon la revendication 3, dans lequel la valeur normalisée pour le niveau d'expression de miR-431-5p dans une plage de 0,0 à 0,4 indique VSCC, et/ou la valeur normalisée pour le niveau d'expression de miR-431-5p dans une plage de 0,5 à 1,3 indique VIN.

6. Procédé selon la revendication 3, dans lequel la valeur normalisée pour le niveau d'expression de miR-630 dans une plage de 0,0 à 0,15 indique VSCC, et/ou la valeur normalisée pour le niveau d'expression de miR-630 dans une plage de 0,16 à 0,6 indique VIN.

7. Procédé selon la revendication 3, dans lequel la valeur normalisée pour le niveau d'expression de miR-23b-3p dans une plage de 0,35 à 1,3 indique VSCC, et/ou la valeur normalisée pour le niveau d'expression de miR-23b-3p dans une plage de 0,1 à 0,34 indique VIN.

8. Procédé selon la revendication 3, dans lequel la valeur normalisée pour le niveau d'expression de miR-23b-3p dans une plage de 1,8 à 4,8 indique un sujet sain, et/ou la valeur normalisée pour le niveau d'expression de miR431-5p dans une plage de 1,4 à 2,6 indique un sujet sain, et/ou la valeur normalisée pour le niveau d'expression de miR-630 dans une plage de 0,7 à 1,1 indique un sujet sain.

9. Procédé selon l'une quelconque des revendications précédentes, dans lequel les biomarqueurs sélectionnés sont miR-222-3p et miR-431-5p et sont utilisés ensemble.

10. Procédé d'identification d'un ou plusieurs biomarqueurs pour VIN ou VSCC chez un sujet, comprenant les étapes consistant à :

    - mesurer le niveau d'expression d'au moins un biomarqueur candidat dans un échantillon du sujet testé,
    - mesurer le niveau d'expression d'un normalisateur dans l'échantillon du sujet testé, la combinaison de hsa-miR-93-5p et hsa-miR-425-5p étant utilisée comme normalisateur,
    - normaliser le niveau d'expression mesuré d'au moins un dit biomarqueur par rapport au normalisateur pour obtenir la valeur normalisée du niveau d'expression d'au moins un dit biomarqueur,

- comparer le niveau d'expression normalisé avec un niveau d'expression de référence normalisé, dans lequel la comparaison dudit niveau d'expression normalisé d'au moins un dit biomarqueur avec ledit niveau d'expression de référence normalisé est indicative pour déterminer si le biomarqueur candidat est un biomarqueur pour VIN ou VSCC,

dans lequel ledit niveau d'expression de référence est une ou plusieurs valeurs établies à partir d'un ou plusieurs échantillons biologiques,

et dans lequel l'échantillon est du sang total ; une fraction sanguine telle que le plasma, le sérum, les fractions de cellules sanguines ou les plaquettes sanguines; de préférence une fraction sanguine, plus préférablement du plasma sanguin.

**Fig.1.** Expression levels of four selected miRNAs in the plasma samples of VIN and VSCC patients.

**Fig. 2** Expression levels of four selected miRNAs in the plasma samples of VIN - as divided into HSIL and dVIN subtypes - and VSCC patients.

**Fig. 3** Receiver operating characteristics (ROC) curve based on the normalized expression levels of miR-222-3p and miR-431-5p.

VSCC − VIN, 2 miRNA, auc=0.985

Fig. 4 Expression levels of four selected miRNAs in plasma samples from the control group of healthy subjects (C), VIN and VSCC patients.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009075258 A1
- WO 2013022786 A2

**Non-patent literature cited in the description**

- **XIUHUA YANG et al.** miRNA expression profile of vulvar squamous cell carcinoma and identification of the oncogenic role of miR-590-5p. *ONCOLOGY REPORTS,* 22 October 2015, vol. 35 (1), ISSN 1021-335X, 398-408
- **BEATRIZ MAIA et al.** MiR-223-5p works as an oncomiR in vulvar carcinoma by TP63 suppression. *Oncotarget,* 09 November 2014
- **V. AMBROS et al.** *RNA,* 2003, vol. 9 (3), 277-279
- WHO Classification of Tumours of Female Reproductive Organs WHO/IARC Classification of Tumours. 2014, vol. 6
- **VÁRALLYAY E ; BURGYÁN J.** *Nat Protoc.,* 2008, vol. 3 (2), 190-6
- **CHANG-GONG LIU CG.** *Nature Protocols,* 2008, vol. 3, 563-578
- **TAM S ; DE BORJA R ; TSAO MS ; MCPHERSON JD.** *Laboratory Investigation,* 2014, vol. 94, 350-358
- **URBANEK MA ; NAWROCKA AU ; WJ.** *Int J Mol Sci.,* 2015, vol. 16 (6), 13259-13286
- **SUN Y ; GREGORY KJ ; CHEN NG ; GOLOVLEV V.** *Analytical Biochemistry,* 2012, vol. 429 (1), 11-17
- **JONES G ; BARKER A.** *Clin Biochem Rev,* 2008, vol. 29 (1), S93-S97
- **JONES GR ; BARKER A ; TATE J ; LIM CF ; ROBERTSON K.** *Clin Biochem Rev.,* 2004, vol. 25 (2), 99-104
- **BORNSTEIN J ; BOGLIATTO F ; HAEFNER HK ; STOCKDALE CK ; PRETI M ; BOHL TG ; REUTTER J.** Committee IT: The 2015 international society for the study of vulvovaginal disease (issvd) terminology of vulvar squamous intraepithelial lesions. *J Low Genit Tract Dis,* 2016, vol. 20, 11-14
- **JI T ; ZHENG ZG ; WANG FM ; XU U ; LI LF ; CHENG QH ; GUO JF ; DING XF ; VASUDEVAN S.** Differential microrna expression by solexa sequencing in the sera of ovarian cancer patients. *Asian Pac J Cancer Prev,* 2014, vol. 15, 1739-1743
- **V. AMBROS et al.** A uniform system for microRNA annotation. *RNA,* 2003, vol. 9 (3), 277-279
- **VÁRALLYAY E ; BURGYÁN J ; HAVELDA Z.** MicroRNA detection by northern blotting using locked nucleic acid probes. *Nat Protoc.,* 2008, vol. 3 (2), 190-6
- **CHANG-GONG LIU ; GEORGE ADRIAN CALIN ; STEFANO VOLINIA ; CARLO M CROCE.** MicroRNA expression profiling using microarrays. *Nature Protocols,* 2008, vol. 3, 563-578
- **SHIRLEY TAM ; RICHARD DE BORJA ; MING-SOUND TSAO ; JOHN D MCPHERSON.** Robust global microRNA expression profiling using next-generation sequencing technologies. *Laboratory Investigation,* 2014, vol. 94, 350-358
- **MARTYNA O. URBANEK ; ANNA U. NAWROCKA ; WLODZIMIERZ J. KRZYZOSIAK.** Small RNA Detection by in Situ. *Hybridization Methods Int J Mol Sci.,* June 2015, vol. 16 (6), 13259-13286
- **YESUNKALVIN J. ; GREGORYNELSON G.** Chen-ValGolovlev Rapid and direct microRNA quantification by an enzymatic luminescence assay. *Analytical Biochemistry,* 01 October 2012, vol. 429 (1), 11-17
- **GRAHAM JONES ; ANTONY BARKER.** Reference Intervals. *Clin Biochem Rev,* August 2008, vol. 29 (1), S93-S97
- **GRAHAM RD JONES ; ANTONY BARKER ; JILL TATE ; CHEN-FEE LIM ; KEN ROBERTSON.** The Case for Common Reference Intervals. *Clin Biochem Rev.,* May 2004, vol. 25 (2), 99-104
- **XIE F ; XIAO P ; CHEN D et al.** miRDeepFinder: a miRNA analysis tool for deep sequencing of plant small RNAs. *Plant Mol Biol.,* 31 January 2012
- **VANDESOMPELE J ; DE PRETER K ; PATLYN F et al.** Accurate normalization of real-time quantitative RT-PCR data by geometric averaging of multiple internal control genes. *Genome Biol,* 2002, vol. 3
- **MANNOOR K ; LIAO J ; JIANG F.** Small nucleolar RNAs in cancer. *Biochim Biophys Acta,* 2012, vol. 1826 (1), 121-8